# EUROPEAN PATENT APPLICATION

(11) **EP 2 842 583 A1**
(43) Date of publication of application: **04.03.2015**
(21) Application number: 13182122.5
(22) Date of filing: 29.08.2013
(51) Int. Cl.: A61L 31/06, A61L 31/14, C08G 18/48, C08G 18/66, C08G 18/73, C08G 18/12, C08G 18/24, C09D 175/08, C08G 18/32, C08G 18/40, C08G 18/42, C08G 63/664

(54) **Biodegradable and bioerodible polyurethanes, method of preparation thereof and use thereof**

(71) Applicant: Ella-CS, s.r.o., 506 06 Hradec Králové (CZ); GP Novatech s.r.o., 339 01 Klatovy (CZ)
(72) Inventor: Novak, Pavel, 339 01 Klatovy (CZ)
(74) Representative: Hartvichova, Katerina

(57) **Abstract**

The present invention relates to biodegradable and bioerodible polyurethanes with a controlled degradation time and the use thereof in medicine, in particular as stent covers. The polyurethanes are composed of substantially hydrophobic block ABA poly(ester-ether) diols, hydrophilic polyoxyalkylene polymer, diisocyanate and a low-molecular polyol.

## Description

### Field of Art

The present invention relates to biodegradable and bioerodible polyurethanes with controlled degradation time and their use in medicine, e.g. as covers for stents in gastrointestinal tract.

### Background Art

Biodegradable materials are broadly used in medicine as devices for temporary connection or covering of tissues or for their temporary replacement. Synthetic surgical fibers are a representative biodegradable material that has been used for a long time. Polyesters derived from hydroxyalkylcarboxylic acids, namely glycolic, lactic, γ-butyric and ε-caproic acids, both homo- and co-polymers, are very often used as synthetic biodegradable materials. The polymerization degree of these polymers reaches the order of magnitude from thousand to tens of thousands. An overview of biodegradable polyesters can be found in: Williams S.F., Martin D.P., 2005. Application of Polyhydroxyalkanoates (PHA) in Medicine and Pharmacy. Biopolymers Online.

Polyesters with a low polymerization degree of units to hundreds, so called oligomers, are utilized as a component of the chains of more complex biodegradable polymers, polyurethanes e.g.

Low temperature water soluble biodegradable polymers containing hydrophobic polyester blocks and hydrophilic blocks from polyoxyethylene or ethylene oxide-propylene oxide copolymer are described in US patents US 8,211,959; US 5,702,717; US 6,004,573; US 6,117,949. These polymers form a gel at body temperature and thus can be used for controlled drug release.

Patent US 5,514,380 describes thermoplastic biodegradable multiblock polymers with hydrophilic polyoxyethylene or ethylene oxide-propylene oxide copolymer blocks and hydrophobic crystallizable and non-swellable polyester block forming a hard domain consisting of polylactide, polyglycolide, polylactideglycolide and polycaprolactone. These biomedical polymers have been developed as a drug delivery matrix.

Patent US 5,708,073 describes polyurethane compositions containing specified amounts of ionic or acidic groups and specified amounts of polyester groups and, in case of hydrophilic compositions, also polyoxyethylene groups. These compositions are base-degradable and they are intended as laminating adhesives that allow removal of the polymer and recovery of the associated substrate, e.g., aluminum. They can also be used for insecticide or herbicide encapsulation.

In WO99/64491, biodegradable linear block polyurethanes are described, containing soft polyester segments composed of lactide, glycolide, butyrolactone, and caprolactone homoor co-polymers and hard blocks are from 1,4-butylene diisocyanate and 1,2-ethylene diol, 1,4-butylene diol or 1,6-hexylene diol.

Insoluble cross-linked biodegradable polyurethanes are described in several patents. In the patent US 6,555,645, e.g., polyurethane resin is described which is composed of 2,5-/2,6-diisocyanatomethylbicyclo[2.2.1]heptane, saccharides, aliphatic polyester polyol and polyhydroxykarboxylate polyol. The intended use is for staples, disc cases or card bases.

Biodegradable polyurethanes that are suitable for reducing adhesions associated with postoperative surgery are described in the patent US 6,136,333. For the manufacture of various products such as film, rod, tube, bead, foam, ring, viscous liquid, dispersion, suspension, viscous solution, spray and gel, chain-extended poly(hydroxy-carboxylic acid)/poly(oxyalkylene) triblock diols are suggested. By poly(oxyalkylene), poly(ethylene oxide) homopolymer or poly(ethylene oxide)-co-poly(propylene oxide) block copolymer is meant and chain extension is performed by diisocyanates.

No biodegradable polymers engineered for degradable stents covering are on the market. There are many demands that a stent cover must fulfill, such as appropriate values of Young's modulus and elongation at break, stability during storage, non-toxicity of the polymer and its degradation products, degradation rate not lower than that of the stent material, good adhesion to the stent material. The aim of the present invention is to provide a material that meets all the requirements for a stent cover.

### Disclosure of the Invention

The present invention provides a linear, branched or cross-linked biodegradable or bioerodible polyurethane, obtainable by the reaction of - a substantially hydrophobic oligomeric diol **D1** of formula HO-ABA-OH, wherein

A is a polyester obtainable by polymerization of 2 to 50 monomer units, which are aliphatic hydroxycarboxylic acid or ester selected from the group consisting of lactic acid, lactide, glycolic acid, glycolide, beta-hydroxy propionic acid, beta-propiolactone, gamma-hydroxy butyric acid, gamma-butyrolactone, delta-hydroxy valeric acid, delta-valerolactone, epsilon-hydroxy caproic acid, epsilon-caprolactone and mixtures thereof, and

B is a polyoxyalkylene polymer of formula (O-CH₂CH₂CH₂CH₂)_{y}, wherein y = 4 to 50, or (0-CH₂CH(CH₃))_{z}, wherein z = 4 to 100;
- an oligomeric diol **D2** of formula H-(O-CH₂CH₂)ₐ OH, wherein a = 3 to 100;
- a low-molecular polyol **D3** selected from the group consisting of ethylene glycol, 1,4-butanediol, 1,6-hexanediol, trimethylolpropane, glycerin, and tetramethylol methane; and
- diisocyanate **DIS** selected from the group consisting of hexamethylene diisocyanate, lysine diisocyanate, cyclohexyl diisocyanate, isophoron diisocyanate and bis(4-isocyanatohexyl) methane.

In a preferred embodiment, the oligomeric diol **D1** has a molecular weight of between 450 Da and 18000 Da, more preferably between 630 and 7000 Da. Preferably, the A-block has between 2 and 50, more preferably between 4 and 20, monomeric units in length; and the B-block preferably varies in molecular weight from about 200 Da to about 6000 Da, more preferably from about 200 Da to about 2500 Da.

The term "molecular weight" refers more specifically to the number average molecular weight.

Preferably, the molar ratio of the sum of hydroxyl groups of **D1**, **D2** and **D3** blocks to isocyanate groups of **DIS** is within the range of 0.85 to 1.15.

Preferably, the molar ratio of diols **D1** and **D2** is within the range of from 0.2 : 1 to 1: 0.2, while the molar ratio of hydroxy groups in blocks (**D1** + **D2**) / **D3** is within the range of 0.1 to 10.0.

Preferably, A is poly(epsilon-caprolactone).

Preferably, **D3** is 1,4-butanediol or 1,6-hexanediol.

Preferably, **DIS** is hexamethylene diisocyanate.

The present invention further provides a method of producing the linear, branched or crosslinked biodegradable or bioerodible polyurethane of the present invention. The components **D1**, **D2**, **D3** and **DIS** are reacted at the temperature within the range of 40 °C to 100 °C, optionally in the presence of an aprotic solvent such as dimethyl formamide, dimethyl sulfide, dimethyl acetamide or diethyl acetamide. The synthesis may be performed in the presence of organometallic or amine type catalyst or mixture of these catalysts. Suitable catalysts for this type of reactions are known in the art, e.g., described in Saunders J.H., Frisch K.C. Polyurethanes. Chemistry and Technology, Part 1. Interscience, New York 1963.

The synthesis of the polyurethane may be performed in one step, where all components react simultaneously, or in two steps. In the two-step synthesis, the first step is a reaction of **D1** and **D2** with **DIS**, and in the second step, **D3** is added. In both cases, linear, branched or cross-linked polyurethane arises depending on the functionality of the low-molecular alcohol.

The present invention further relates to the use of the polyurethane of the present invention for production of medical products, in particular for production of biodegradable stent coating, preferably for production of biodegradable coating for gastrointestinal stents.

Another object of the present invention is stent for medical use, comprising the polyurethane of the present invention, preferably as coating.

In general, biodegradable and bioerodible polymers contain easily cleavable weak chemical bonds in polymer chains that are exposed to the internal body environment. Such bonds in polyurethanes are in particular ester and urethane bonds that relatively easily hydrolyze, resulting in gradual degradation of the polymers within several weeks to months, so that products from biodegradable polymers can be completely resorbed within said time frame.

The composition of the polyurethane of the present invention allows fine-tuning of the degradation properties of the resulting polymer. The increase of the content of **D2** results in the increase of the degradation rate. The cross-linking (when **D3** contains more than two hydroxy groups) results in the decrease of the degradation rate and changes the kinetics of the degradation.

The basis of the invention is the fact that the rate of hydrolysis depends on the accessibility of the ester and urethane bonds for water. By suitably introducing a relatively small amount of hydrophilic water soluble poly(oxyethylene) polyols into polyurethanes, water swelling of the polyurethanes can be controlled and thereby the hydrolyzation rate. The present invention allows to affect the hydrolysis rate significantly without negatively influencing the mechanical properties. It is important that hydrophilic poly(oxyethylene) were not part of the initial polyester block because only when they form separate blocks, it is possible to control polyurethane swelling and elasticity easily and precisely.

### Examples of carrying out the Invention

### ABA oligomer synthesis

### Example 1

150 g of poly(oxypropylene) diol of the molecular weight of 400 Da was dried by azeotropic distillation with 150 mL of benzene for 3 hours. Then, benzene was distilled off and the poly(oxypropylene) diol was evacuated at the pressure of 150 Pa at 60 °C for 4 hours. Water content of the dry product was 51 ppm. Epsilon-caprolactone was purified by distillation with calcium hydride.

100 g of poly(oxypropylene) diol was mixed with 400 g of purified epsilon-caprolactone and 800 mg of tin(II) 2-ethylhexanoate. The mixture was polymerized under argon at 150 °C for 8 hours. OH group content determined by acetylation method was 0.94 mmol/g. The product was designated "PC".

### Example 2

150 g of poly(tetramethylene oxide) diol of the molecular weight of 650 Da was dried in the same way as described in example 1. Water content of the dry product was 45 ppm. 100 g of poly(tetramethyleneoxide) diol was mixed with 213 g of purified epsilon-caprolactone and 800 mg of tin(II) 2-ethylhexanoate. The mixture was polymerized under argon at 150 °C for 8 hours. OH group content determined by acetylation method was 0.98 mmol/g. The product was designated "TC".

### Polyurethane synthesis

### Example 3

7.87 g of TC, 0.17 g of vacuum-dried poly(oxyethylene) diol (POE) having the molecular weight of 1500 Da and 0.44 g of 1,6-hexane diol were heated to 60 °C and stirred for 10 minutes. Then, 1.30 g of hexamethylene diisocyanate and 0.01 ml of a 0.1 mol/l solution of dibutyl tin(II) dilaurate in toluene as a catalyst was added and while stirring the temperature was raised to 80°C. After 2 hours, the product was cooled down and chloroform was added to make 5% solution by weight. The solution was vacuum-filtred through S1 sintered glass and polyurethane foils of 0.2 mm thickness were prepared by solvent evaporation.

### Example 4

Polyurethane was prepared in the same manner as described in the Example 3 from 9.01 g of TC, 2.65 g of POE having the molecular weight of 1500 Da, 0.25 g of 1,4-butane diol and 1.48 g of hexamethylene diisocyanate.

### Example 5

Polyurethane without poly(oxyethylene) diol was prepared in the same manner as described in the Example 3 from 10.00 g of TC, 0.44 g of 1,4-butane diol and 1.65 g of hexamethylene diisocyanate.

### Example 6

Polyurethane was prepared in the same manner as described in the Example 3 from 7.83 g of TC, 1.14 g of POE having the molecular weight of 1500 Da, 0.27 g of 1,4-butane diol and 1.30 g of hexamethylene diisocyanate.

### Example 7

7.83 g of PC, 1.14 g of vacuum-dried poly(oxyethylene) diol (POE) of the molecular weight of 1500 Da were dissolved in 100 ml of dried dimethyl acetamide. 1.30 g of hexamethylene diisocyanate and 0.01 ml of a 0.1 mol/l solution of dibutyl tin(II) dilaurate in toluene as a catalyst were added and the mixture was reacted for 60 minutes while stirring at 60°C. Then, 0.27 g of 1,4-butane diol was added, the temperature was raised to 80 °C and the reaction was carried out for another 2 hours. The mixture was cooled down, vacuum-filtred through S1 sintered glass and the polyurethane was percipitated by adding distilled water. The precipitate was dried in the air and in vacuum of 10 Pa at 40 °C for 5 hrs. Then it was dissolved in chloroform to make 5% (w/w) solution. Polyurethane foils of 0.2 mm thickness were prepared by chloroform evaporation.

### Example 8

9.00 g of PC, 0.28 g of vacuum-dried poly(oxyethylene) diol (POE) of the molecular weight of 600 Da and 0.41 g of trimethylol propane were heated to 70 °C and stirred for 10 minutes. Then, 2.48 g of bis(4-isocyanatohexyl) methane and 0.01 ml of a 0.1 mol/l solution of dibutyl tin(II) dilaurate in toluene as a catalyst was added, the mixture was stirred for another 2 minutes, degassed and then it was filled in the mold consisting of two concentric polypropylene cylinders with 1 mm gap in between. The mold was placed into an oven heated to the temperature of 80°C. After 2 hours, the mold was taken out, cooled down, disassembled and a transparent elastic tube with 10 mm inner diameter and 1 mm wall thickness was obtained.

### Example 9

2.5 g of polyurethane described in the example 3 and 2.25 g of TC were dissolved in 95 g of chloroform. Braided esophagus stent from polydioxanone was covered by 0,2 mm coating by chloroform evaporation from the solution. TC was added in order to control elasticity of the stent cover and to support degradation (plasticizer).

### Example 10: Polyurethane testing

Swelling of the polyurethane was determined as weight increase after 24 hrs of maintaining the sample in a phosphate-buffered saline of pH = 7.3.

Degradation of polyurethanes was performed in a phtalate buffered solution of pH 3 at 87 °C. This solution imitates gastric juice. Elevated temperature speeds up degradation 32 times compared to body temperature.

Elongation at break and Young's modulus were used as criteria for degradation. During degradation, the polymer chains are cleaved and the Young's modulus and elongation at break drop. "Dog bone" samples were cut from the dry polyurethane foil for determination of elongation at break.

The values of both elongation at break and Young's modulus clearly show that increasing in water content in the sample line 5 < 3 < 7 < 6 < 4 speeds up degradation. The water content is controlled by the amount of D2. On the other hand, if the polyurethane is crosslinked (Example 8), the degradation is slowed down markedly. Thus, a big variety of polyurethanes can be prepared with "custom-tailored" mechanical properties and degradation time.

Characteristics of polymers are summarized in the following table:

| Example | Water content in mass % | Eb(0) in % | Eb (24) in % | Eb (48) in % | E (0) in MPa | E (24) in MPa | E (48) in MPa |
|---|---|---|---|---|---|---|---|
| 5 | 1,4 | 1880 | 740 | 120 | 7,4 | 3,9 | 0,8 |
| 3 | 2,5 | 2140 | 550 | 40 | 6,4 | 3,5 | 0,5 |
| 7 | 7,6 | 760 | 55 | immeasurable | 7,5 | 2,7 | immeasurable |
| 6 | 9,8 | 2450 | 160 | immeasurable | 6,9 | 2,2 | immeasurable |
| 4 | 27,6 | 1270 | immeasurable | immeasurable | 5,9 | immeasurable | immeasurable |
| 9 | 18,4 | 1910 | 40 | immeasurable | 6,2 | 2,4 | immeasurable |
| 8 | 2,0 | 450 | 220 | 390 | --- | --- | --- |

| | | | | | | | |
|---|---|---|---|---|---|---|---|
| Eb(0) / E(0) Elongation at break / Young's modulus of a fresh sample; Eb (24) / E(24) Elongation at break/ Young's modulus of a sample that was degraded for 24 hrs in a phtalate buffered solution of pH = 3 at 87°C; Eb (48) / E(48) Elongation at break / Young's modulus of a sample that was degraded for 48 hrs in a phtalate buffered solution of pH = 3 at 87°C; "Immeasurable" means that the sample was altered (degraded) to such extent that it was not possible to mount it into clamps. | | | | | | | |

### Industrial applicability

Biodegradable and bioerodible polyurethanes according to the present invention have suitable mechanical and chemical properties for medical use and they can readily be prepared. Soluble polyurethanes can be processed by well-known methods to final products in the form of foils, tubes or other.

Final products in the form of foils, tubes or other from non-soluble cross-linked polyurethanes can be produced by casting into metal or plastic, e.g. polypropylene, molds.

## Claims

1. A linear, branched or cross-linked biodegradable or bioerodible polyurethane obtainable by the reaction of
- a substantially hydrophobic oligomeric diol **D1** of formula HO-ABA-OH, wherein
A is a polyester obtainable by polymerization of 2 to 50 monomer units, which are aliphatic hydroxycarboxylic acid or ester selected from the group consisting of lactic acid, lactide, glycolic acid, glycolide, beta-hydroxy propionic acid, beta-propiolactone, gammahydroxy butyric acid, gamma-butyrolactone, delta-hydroxy valeric acid, deltavalerolactone, epsilon-hydroxy caproic acid, epsilon-caprolactone and mixtures thereof, and
B is a polyoxyalkylene polymer of formula (O-CH₂CH₂CH₂CH₂)_{y}, wherein y = 4 to 50, or (0-CH₂CH(CH₃))_{z}, wherein z = 4 to 100;
- an oligomeric diol **D2** of formula H-(O-CH₂CH₂)ₐ OH, wherein a = 3 to 100;
- a low-molecular polyol **D3** selected from the group consisting of ethylene glycol, 1,4-butanediol, 1,6-hexanediol, trimethylolpropane, glycerin, and tetramethylol methane; and
- diisocyanate **DIS** selected from the group consisting of hexamethylene diisocyanate, lysine diisocyanate, cyclohexyl diisocyanate, isophoron diisocyanate and bis(4-isocyanatohexyl) methane.

2. The polyurethane according to claim 1, wherein the oligomeric diol **D1** has a molecular weight of between 450 Da and 18000 Da, the A-block has between 2 and 50 monomeric units in length and the B-block varies in molecular weight from about 200 Da to about 6000 Da.

3. The polyurethane according to any preceding claim, wherein the molar ratio of the sum of hydroxyl groups of **D1**, **D2** and **D3** compounds to isocyanate groups of **DIS** is within the range of 0.85 to 1.15.

4. The polyurethane according to any preceding claim, wherein the molar ratio of diols **D1** and **D2** is within the range of from 0.2 : 1 to 1: 0.2, while the molar ratio of hydroxy groups in blocks (**D1** + **D2**) / **D3** is within the range of 0.1 to 10.0.

5. A method of producing the polyurethane according to any of the preceding claims, wherein the components **D1**, **D2**, **D3** and **DIS** are reacted at the temperature within the range of 40 °C to 100 °C, optionally in the presence of an aprotic solvent and optionally in the presence of organometallic or amine type catalyst or mixture of these catalysts.

6. The method of claim 5, wherein the reaction is performed in two steps, whereas the first step is a reaction of **D1** and **D2** with **DIS**, and in the second step, **D3** is added.

7. The method of claim 5, wherein the reaction is performed in one step, where all components react simultaneously.

8. Use of the polyurethane according to any of claims 1 to 4 for production of medical products, in particular for production of biodegradable stent coating.

9. A stent for medical use, comprising the polyurethane of any of claims 1 to 4.
